# EUROPEAN PATENT SPECIFICATION

(11) **EP 2 105 097 B1**
(45) Date of publication and mention of the grant of the patent: **23.03.2011**
(21) Application number: 08153423.2
(22) Date of filing: 27.03.2008
(51) Int. Cl.: A61B 17/00

(54) **Apparatus for removing blood vessels from a patient's body**
Vorrichtung zum Entfernen von Blutgefäßen aus einem Patientenkörper
Appareil pour enlever des vaisseaux sanguins du corps d'un patient

(43) Date of publication of application: 30.09.2009
(73) Proprietor: Oesch, Andreas, 3011 Bern (CH)
(72) Inventor: Oesch, Andreas, 3011 Bern (CH)
(74) Representative: BOVARD AG

(56) References cited:
- EP-A- 0 605 351
- WO-A-01/62137
- WO-A-2007/017561
- US-A1- 2005 283 174

## Description

### Technical Field of the Invention

The present invention relates to a surgical apparatus for removing blood vessels from a patient's body. In particular, the present invention relates to a surgical apparatus for removing a blood vessel from a patient's body, comprising an elongated member, having a front end for inserting the elongated member into, and pushing it through, the blood vessel, and a rear end for pulling the elongated member out of the blood vessel. Such surgical apparatus are often referred to as "strippers."

### Background Art

Varicose veins, usually in the legs (and in particular on the calves), but also in other parts of the body, represent a very common condition. Varicose veins tend to be more common in older women, but in fact affect about 30% of the world's adult population at some point in life.

Basically, varicose veins are veins that have become enlarged and twisted. This occurs in general when the vein wall weakens, causing a vein to dilate. In the circulatory system, veins in general carry low-oxygen blood towards the heart. For this purpose, veins have leaflet valves to prevent blood from flowing in the wrong direction (back down the legs), while leg muscles are in charge of pumping the veins in order to return blood to the heart. If veins become dilated, the leaflets of the valves no longer meet properly, and these valves do not work anymore.

The superficial veins of the legs, which are subject to high pressure when standing, commonly become varicose. Besides being inconvenient for cosmetic reasons, varicose veins are often painful, especially when standing or sitting. Moreover, in later stages, the impaired circulation may lead to leg swelling, itching, skin changes and even ulcers.

Conventionally, one of the most common treatments for varicose veins is their complete removal (often referred to as "stripping"). As most of the blood in the legs is returned to the heart by the deep veins, and the superficial veins only return about 10% of the blood volume, the superficial veins can be removed without any serious harm. In the conventional stripping technique, the surgeon makes two incisions, e.g. in the ankle and in the groin region, inserts a special wire into the vein, and advances the wire through the vein until reaching the other incision. Then, the vein is tied to the wire which is then pulled backwards and taken out of the leg.

Two methods can be distinguished: a so-called "exostripping" and the opposite technique "endostripping." The exostripping comprises pulling the vein longitudinally out of the patient's body, whereby a stopper element causes the vein to collapse in an accordion-fold manner. In this technique, there is an elevated risk of injuries to the surrounding tissues caused by the stopper element. In contrast, in the endostripping technique, the vein is invaginated, i.e. the end of the vein which is tied to the wire is pulled out through the hollow inner portion of the vein itself. In other words, the vein is turned "inside out" and then stripped out of the leg. The advantage of this endostripping technique is that fewer tissue injuries occur outside of the vessel being stripped.

Anaesthesia is generally required for surgical operations on varicose veins. Conventionally, vein stripping has been performed under general anaesthesia. Along with more modern stripping techniques, use of regional or local anaesthesia has been introduced which, owing to fewer side effects, is preferred by an increasing number of surgeons.

The so-called tumescent anaesthesia is a further development of local anaesthesia. Tumescent anaesthesia is commonly used in cosmetic surgery (especially liposuction), and consists basically of a voluminous subcutaneous infiltration of a special anaesthetic solution which causes the surrounding tissue to swell. For this purpose, very large volumes of highly diluted anaesthetic agents are required which have to be applied in the immediate proximity of the blood vessel to be removed. However, a very precise application of anaesthetics (required by the tumescent anaesthesia, but also by conventional local anaesthetic methods) may be difficult on deeper lying veins, unless diagnostic ultrasound devices are used. Moreover, the anaesthetic agents are commonly applied in the operation region of the patient's body by means of conventional subcutaneous injections or similar methods in a step preceding the actual removal of the blood vessel. This makes the surgery more complicated, and requires additional instruments and specialists.

US 2005/283174 discloses a surgical apparatus for removing a blood vessel according to the preamble of claim 1.

### Disclosure of Invention

It is thus an object of this invention to propose a new and improved apparatus for removing blood vessels from a patient's body that do not present the above-mentioned drawbacks of the prior art. It is in particular an object of this invention to propose a new apparatus for removing blood vessels from a patient's body which enables dispensing of an anaesthetic agent simultaneously and at the very place where the removing of the blood vessel takes place, whereby a particularly gentle and minimally invasive surgical technique is used.

According to the present invention, this and other objects are achieved in particular through the features of the independent claims. In addition, further advantageous embodiments follow from the dependent claims and the description.

In particular, this object is achieved through the invention in that in a surgical apparatus for removing a blood vessel from a patient's body, the apparatus comprising an elongated member, having a front end for inserting the elongated member into and pushing it through the blood vessel, a rear end for pulling the elongated member out of the blood vessel, a hollow member for conducting a fluid, which hollow member is attached to the rear end of the elongated member, and at least one aperture in fluid connection with the hollow member is provided on the surgical apparatus, for dispensing the fluid during the removal of the blood vessel. The front end of the apparatus is further arranged to be pushed through a wall of the vessel.

This invention has the advantage, among others, that it permits an anaesthetic agent or any similar fluid to be provided simultaneously and precisely to the surrounding tissues of the blood vessel during the surgical operation without the help of additional instruments and technologies. Moreover, the special design of the surgical apparatus according to the present invention also enables the precise removal of a preoperatively defined blood vessel segment without the necessity of surgical exposure of the blood vessel itself and makes a removal of the blood vessel much less traumatising. In a very special embodiment, the hollow member according to this invention can be removably attached to the rear end of the elongated member, such that the surgical apparatus can, if required, be used in the same way as a classical apparatus, without availing of all the advantages which the apparatus according to this invention offers. In this case, a suitable connecting means has to be provided which can allow the hollow member to be pulled upon, without the attached hollow member becoming loose or coming off.

In an embodiment variant, the at least one aperture for dispensing the fluid during the removal of the blood vessel is provided on the hollow member. The advantage of this embodiment variant is, inter alia, that the fluid can be dispensed on the rear end of the elongated member, thus making it possible to provide the anaesthetic or rinsing agent to the region of the patient's body in which the blood vessel has already been removed. In this way, the removal can be made even less traumatising. In addition, as the apertures for dispensing the fluid are provided on the hollow member itself, any conventional elongated member can, in general, be used in connection with a hollow member according to the invention.

In another embodiment variant, at least one interface element for connecting a fluid reservoir and filling the fluid into the hollow member is provided on the hollow member. This interface element can e.g. preferably be located at the free end of the hollow member such that a fluid reservoir can be connected to the hollow member in an easy way. This interface element can be adapted to match perfectly a syringe extremity such that a commonly used anaesthetic reservoir can be used. The advantage of this embodiment variant resides in the fact that, among other things, a number of different fluid reservoirs can be connected to the hollow member. If various interface elements are used, different fluids can be provided to the hollow member alternatively or at the same time, which can simplify or accelerate the surgical operations.

In a further embodiment variant, the front end of the elongated member is curved and/or tapered laterally. The advantage of this embodiment variant is, inter alia, that the surgical operations can be simplified. For example, when the laterally tapered shape of the front end of the elongated member is used, perforation of the blood vessel's wall, which is necessary for the removal, can be facilitated. On the other hand, a curved shape of the front end of the elongated member facilitates the insertion and, particularly, the pushing of the elongated member through the blood vessel to be removed.

In another embodiment variant, an orifice is provided on the front end of the elongated member. The advantage of this embodiment variant is, inter alia, that the surgical apparatus according to this embodiment variant can be used to remove the blood vessel according to the endostripping (invaginating) technique. For this purpose, an extremity of the blood vessel to be removed can be attached to the front end of the elongated member using the orifice. When the elongated member is pushed through the blood vessel to the desired point, a small skin incision can be made exactly above the front end. The blood vessel can thereby be easily localised, divided and fixed to the front end of the elongated member, which is pulled back again. In doing so, the blood vessel is turned inside out, and is pulled out together with the apparatus from the patient's body. Simultaneously, the anaesthetic and/or rinsing agent can be dispensed through the hollow member to the portion of the patient's body from which the blood vessel has been removed.

In a further embodiment variant, the elongated member comprises at least one curve. The advantages of this embodiment variant are, among other things, that the inserting of the elongated member into the blood vessel and the pushing of the elongated member through the blood vessel can be facilitated with this particular shape of the elongated member, which follows the shape of the patient's limbs.

In another embodiment variant, the surgical apparatus comprises a projecting member, the blood vessel being crumpled by the projecting member during insertion and pushing through of the elongated member in the blood vessel. The particular advantage of this embodiment variant resides in the fact that the surgical apparatus according to this embodiment variant makes it possible to use a different operating technique. In this case, the elongated member is inserted into and pushed through the blood vessel to be removed. As the projecting member is, in general, much bigger than the blood vessel itself (with respect to the radial dimension), once the entrance of the blood vessel is reached, the blood vessel is crumpled and pushed towards its end by the projecting member. In this situation, the blood vessel is crumpled in an accordion-like manner. When the front end of the elongated member reaches the end of the blood vessel, the blood vessel can be pulled out through this incision. Again, the anaesthetic and/or rinsing agent can be dispensed through the hollow member to the portion of the patient's body from which the blood vessel has been removed simultaneously with the removal itself.

In yet another embodiment variant, the at least one aperture is provided on the projecting member. The advantage of this embodiment variant is, inter alia, that the anaesthetic agent can be dispensed at the very place where the removal of the blood vessel takes place, and not just at the places where the removal has already taken place. It is thus possible to anaesthetise the portion of the patient's body surrounding the blood vessel some instants before the removal takes place in a very easy and precise way.

In a further embodiment variant, the projecting member comprises at least one fluid channel for fluid connection between the hollow member and the at least one aperture. This embodiment variant has the advantage, inter alia, that the hollow member can simply be connected to the one end of the projecting member, without any particular fluid connections. In this way, the same hollow member can be used with all elongated members according to the invention (i.e. both those with and those without the projecting member).

In still another embodiment variant, the cross section of the projecting member perpendicular to the insertion direction has a substantially oval form. This embodiment variant has the advantage, among other things, that it can advantageously follow the anatomical structure of the portion of patient's body surrounding the blood vessel, which can minimise the danger of painful lesions caused by the projecting member during a surgical operation.

In yet a further embodiment variant, the cross section of the projecting member parallel to the insertion direction has a substantially curved and/or tapered form. The advantages of this embodiment variant are, inter alia, that the insertion of the elongated member into the blood vessel is facilitated. Moreover, the danger of inadvertent lesions of deeper tissue structures in the patient's body can be further minimised. Finally, the direction of the curve at the front end of the elongated member can also be visualised at its rear end, as both curves lie substantially in the same plane. In this way, the insertion and the pushing through of the elongated member can be controlled more easily by the surgeon.

In another embodiment variant, the surgical apparatus is made at least partially of plastic. The advantage of this embodiment variant resides in the fact that, among other things, plastic is a relatively cheap material which has however a number of useful properties (i.e. flexibility, hypoallergenic properties, etc.). In particular, surgical apparatus made of plastic can also be used just once and then disposed of.

In a still another embodiment variant, the fluid is an anaesthetic, a saline solution, a vasoconstrictive agent and/or a combination of these. The advantage of this embodiment is, among other things, that the surgical operations can be simplified in a drastic way since one single instrument can be used for the preparation, the operation itself, and the postoperative treatment.

### Brief Description of Drawings

The present invention will be explained in more detail, by way of example, with reference to the drawings in which:
Figure 1 is a perspective representation of a surgical apparatus for removing blood vessels from a patient's body according to a first embodiment of the present invention;
Figure 2 is an enlarged perspective representation of the front end of the surgical apparatus for removing blood vessels from a patient's body according to the first embodiment of the present invention;
Figure 3 is an enlarged perspective representation of the rear end of the surgical apparatus for removing blood vessels from a patient's body according to the first embodiment of the present invention;
Figures 4A and 4B are, respectively, a side view and a view from above of the rear end of the surgical apparatus for removing blood vessels from a patient's body according to the first embodiment of the present invention;
Figure 5 is an enlarged perspective representation of the rear end of the surgical apparatus for removing blood vessels from a patient's body according to a second embodiment of the present invention;
Figures 6A and 6B are, respectively, a side view and a view from above of the rear end of the surgical apparatus for removing blood vessels from a patient's body according to the second embodiment of the present invention.

### Description of Specific Embodiments of the Invention

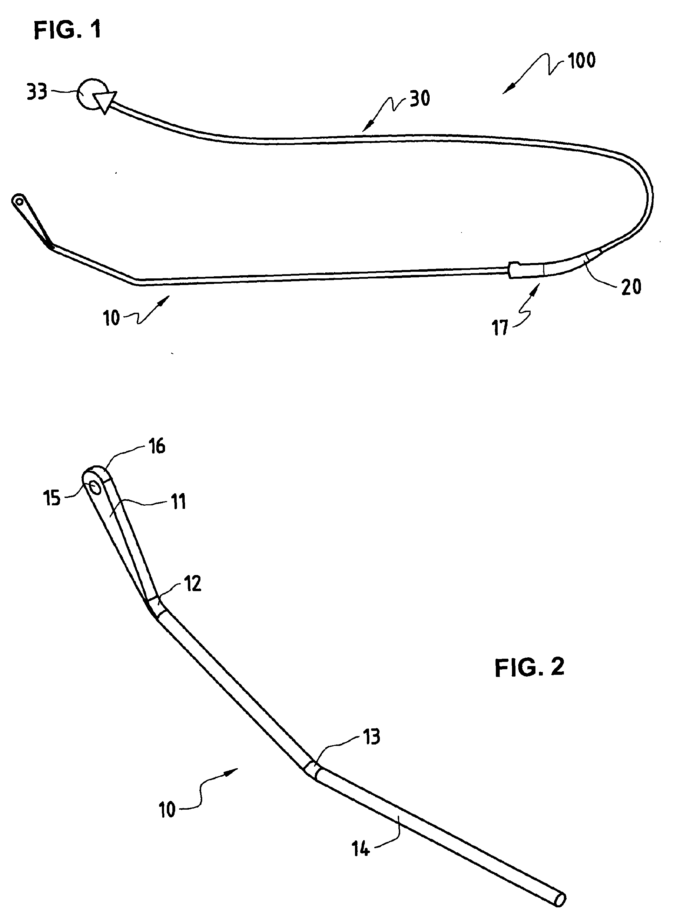

Figure 1 shows a surgical apparatus 100 for removing blood vessels from a patient's body according to a first embodiment of the present invention. The blood vessels to be removed are usually veins, but this invention is clearly not limited to the removal of veins only, and can be used for removing any other blood vessel.

As can be seen in Figure 1, the surgical apparatus 100 according to the first embodiment of this invention comprises an elongated member 10. This elongated member 10 has a front end 16 (represented in more detail in Figure 2) for inserting the elongated member 10 into, and for pushing the elongated member 10 through, the blood vessel to be removed. To this end, an incision is first made in the skin of the patient and in the blood vessel itself; through which the front end 16 is pushed through the blood vessel until the rear end 17 of the elongated body 10 touches the blood vessel within the first skin incision. The blood vessel is fixed here to the elongated body 10. The elongated body is pulled at its front end 16, and the rear end 17 pushes the attached blood vessel in front of it towards the second incision where the blood vessel can be removed from the patient's body.

The elongated member 10 also comprises a rear end 17. This rear end 17 can in particular comprise a projecting member 20. The diameter of this projecting member 20 is bigger in at least one dimension than the diameter of the blood vessel to be removed. When the front end 16 of the elongated member 10 reaches the opposite end of the blood vessel, the front end 16 is pushed through the wall of the blood vessel. A second and very small incision is made precisely above the front end 16. In this way, when the front end 16 of the elongated member 10 is pushed through the blood vessel, the end of the blood vessel in the proximity of the first incision is crumpled by this projecting member 20 of the elongated member 10, in a way similar to the folding of an accordion, and is pushed together with the projecting member 20 towards the second incision. The blood vessel can then be grasped by means of a suitable instrument and pulled out of the patient's body through this second incision. Finally, the surgical apparatus 100, i.e. the elongated member 10, can be pulled back by means of the hollow member 30 and removed from the patient's body through the first incision.

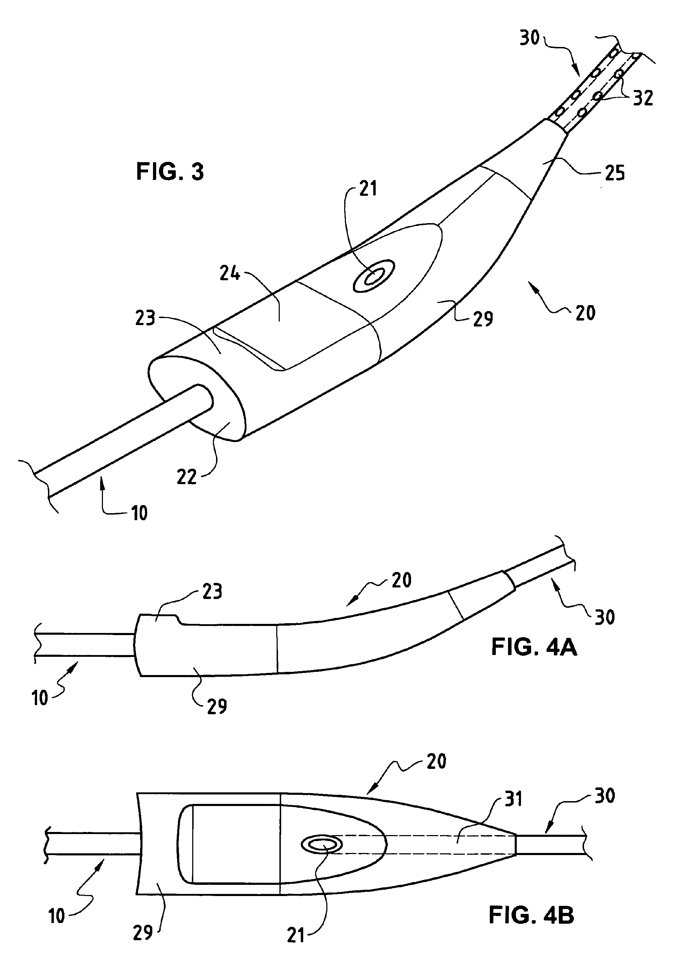

The surgical apparatus 100 according to the present invention further comprises a hollow member 30 for conducting a fluid. This hollow member 30 is attached to the rear end 17 of the elongated member 10, and can be used for pulling the elongated member 10 out of the blood vessel, once the surgical operation has been accomplished. At least one aperture 21, 32 in fluid connection with the hollow member 30 is provided on the surgical apparatus 100 for dispensing the fluid during the removal of the blood vessel (as is shown in more detail in Figures 3, 4, 5 and 6). The fluid which is conducted through the hollow member 30 can in particular comprise an anaesthetic agent, a saline solution, a vasoconstrictive agent, and any other fluid preferably utilised during removal of a blood vessel.

The hollow member 30 in Figure 1 comprises an interface element 33 for connecting a fluid reservoir (not represented) and filling the fluid into the hollow member 30. Having different interface elements for connecting various types of fluid reservoirs is conceivable. These interfaces can also be interchangeable, so as to allow the use of the surgical apparatus 100 according to the invention with different types of fluids and/or fluid reservoirs. In one particular embodiment, different fluid reservoirs can even be attached to the hollow member 30 simultaneously in order to supply the hollow member 30 with different fluids at the same time. Any person skilled in the art will understand that the interface elements 33 can comprise known elements such as valves, cutoffs and other similar elements without departing from the original idea of the invention.

Figure 2 shows the front end 16 of the elongated member 10 of the surgical apparatus for removing blood vessels from a patient's body shown in Figure 1. In order to facilitate pushing the surgical apparatus 100 through the blood vessel, the elongated member 10 preferably comprises at least one curve 12, 13. These curves 12, 13 make it possible to follow the anatomic structure of the tissues surrounding the blood vessel during the insertion of the elongated member 10 and to prevent it from passing into the deep blood vessels, thus reducing the potential lesions of the patient. The curves 12, 13 can be rigid, but can also be designed as articulated joints, thereby allowing better adaptation to different patient anatomies. The elongated member 10 in Figure 1 further comprises a rigid main portion 14 extending from the rear end 17 to the first curve 13 whose dimension can vary, depending on the particular needs of the surgical operation.

The front end 16 of the elongated member 10 in Figure 1 is tapered laterally, and forms a substantially flat portion 11. This substantially flat portion 11 comprises an orifice 15. The orifice 15 can be circular or oval, but can also have any other suitable shape. In such a case, the front end 16 is inserted into the blood vessel to the point to which the vessel should be removed. Here the front end 16 is pushed through the wall of the blood vessel, and a small skin incision can be made exactly above it. The blood vessel can thereby be easily located, divided and fixed to the front end 16 of the elongated member, which is pulled back again. In so doing, the blood vessel is turned inside out, and is pulled out together with the apparatus from the patient's body through the first incision.

In a variation, after the front end 16 of the elongated member 10 has been inserted into the blood vessel through the first incision, the end of the blood vessel which is in the proximity of the first incision is attached to the front end 16 of the elongated member 10. The orifice 15 can preferably be used for this attachment which can be realised using any known technique (i.e. sewing, tying). After the end of the blood vessel has been attached to the front end 16 of the elongated member 10, the blood vessel is invaginated, i.e. its attached end is pushed together with the front end 16 of the elongated member 10 through the hollow portion of the blood vessel towards the other end of the blood vessel and towards the second incision. As in the other embodiment of the present invention, when the front end 16 of the elongated member 10 (with the attached first end of the blood vessel) reaches the opposite end of the blood vessel, a second incision can be made in the blood vessel and in the patient's skin, and the blood vessel can be pulled out of the patient's body through the second incision.

In both cases (i.e. both when the first end of the blood vessel is pushed towards the opposite end of the blood vessel while being crumpled by means of the projecting member 20 or when the first end of the blood vessel is attached to the front end 16 of the elongated member 10 and pushed through the inside of the blood vessel together with the elongated member 10), the apertures 21, 32 allow dispensing the fluid to the tissue surrounding the precise location of the blood vessel's removal, which can guarantee the patient a much less painful and disturbing surgical operation, compared with those carried out by means of common instruments.

The rear end 17 of the surgical apparatus 100 for removing blood vessels from a patient's body according to the embodiment of the present invention shown in Figure 1 is illustrated in Figures 3, 4 and 5. In this embodiment of the invention, the elongated member 10 comprises a projecting member 20. As explained above, this projecting member 20 allows crumpling of the blood vessel to be removed while the front end 16 of the elongated member 10 is being pushed through the blood vessel.

The projecting member 20 has a front portion 23 with a front face 22, where the projecting member 20 is attached to the elongated member 10, and a rear portion 25, where the hollow member 30 is attached to the projecting member 20. It is however also possible to place the projecting member 20 at a position further towards the front end 16 of the elongated member 10, such that the hollow member 30 is not attached directly to the projecting member 20. The front face 22 of the front portion 23 can be flat or curved (as shown in Figures 3, 4A and 4B). Various other profiles and forms of the front face 22 are possible, however. The front face 22 of the front portion 23 has a cross section which is bigger than the cross section of the blood vessel to be removed. Such dimensions of the front face 22 of the front portion 23 of the projecting member 20 are necessary in order to be able to crumple the blood vessel while the front end 16 of the elongated member 10 is being pushed forwards. A substantially flat shape of the front face 22, which reflects the surrounding structures of the blood vessel, makes it possible to receive and support the walls of the blood vessel which is being crumpled and removed, with minimal trauma.

The projecting member 20 comprises a main body 29, which is of a substantially curved form parallel to the insertion direction, as can be seen particularly well in Figure 4A. This curved form of the projecting member 20, together with the curves 12, 13 in the elongated member 10, allows easy insertion of the surgical apparatus 100 into the blood vessel and easy pushing of the surgical apparatus 100 through the blood vessel, with minimal risk of injury to the surrounding tissue. Moreover, the projecting member 20 can have a cross section, perpendicular to the direction of insertion, of substantially oval shape in order to further facilitate the insertion (and also the removal) of the elongated member 10 and to further reduce injuries to the structures surrounding the blood vessel.

The projecting member 20 comprises an aperture 21. The aperture 21 is in fluid connection with the hollow member 30. To this end, a fluid channel 31 is provided in the main body 29 of the projecting member 20, making possible the fluid connection between the hollow member 30 and the aperture 21 (as represented in Figure 4B). It will be evident right away to any person skilled in the art that not only a single aperture 21, but a plurality of apertures 21 can be provided at the projecting member 20. Similarly, a single fluid channel 31 can be replaced by a plurality of fluid channels 31. In this way, the fluid (e.g. an anaesthetic) can be dispensed during the removal of the blood vessel at the very place of the removal. As previously explained, this approach allows surgical operations on blood vessels to be carried out much more easily, since a single surgical apparatus can be used for both administering the anaesthesia and for the actual removal of the blood vessel.

As can be seen in Figure 3, the hollow member 30 can also comprise one or more apertures 32, which can in particular be disposed in the proximity of the projecting member 20 (i.e. of the elongated member 10). The apertures 32 can also be used for dispensing the fluid during the removal of the blood vessels. It is conceivable to let the fluid flow out simultaneously through the aperture 21 provided on the projecting member 20 and the apertures 32 provided on the hollow member 30. It is however also conceivable to block, for whatever reason, the dispensing of the fluid through the aperture 21 or the dispensing of the fluid through the apertures 32 (in this case, particular blocking and/or control means can be provided). In a further conceivable embodiment of the invention, the hollow member 30 can comprise more than one fluid channel, which, in combination with a plurality of interface elements 32, can make it possible to dispense various fluids at the same time. In this case, the aperture (or the apertures) 21 provided on the projecting member 20 might be used for dispensing a first fluid, while the apertures 32 provided on the hollow member 30 might be used for dispensing a second fluid. An inverse situation is also possible of course.

The main body 29 of the projecting member 20 in Figures 3, 4A and 4B comprises a recess 24 in the proximity of the aperture 21. This recess 24 can have smaller or bigger dimensions and various shapes, and facilitates the handling of the instrument and the dispensing of the fluid during the insertion of the surgical apparatus 100 into the blood vessel, creating a small interstice between the main body 29 of the projecting member 20 and the blood vessel's wall. This serves the gripping of the instrument, and the fluid can be brought into the loose tissue without any problem. Once again, shapes for the recess 24 other than that seen in the drawing, and even projecting members without the recess 24 are imaginable.

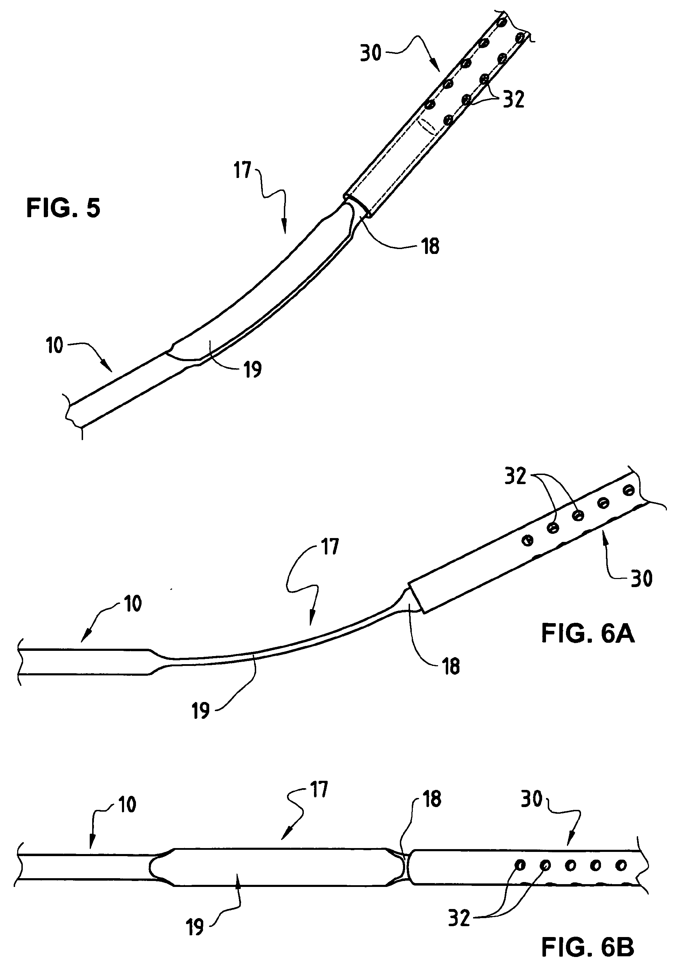

Figures 5, 6A and 6B show the rear 17 end of the surgical apparatus 100 for removing blood vessels from a patient's body according to the second embodiment of the present invention, i.e. the embodiment used with the invagination method for removal of the blood vessels.

As can be seen in figures 5, 6A and 6B, the surgical apparatus 100 does not comprise the projecting member 20, according to this embodiment of the present invention. The hollow member 30 is therefore attached directly to the elongated member 20, in its tail portion 18. In an embodiment variant, the hollow member 30 can simply be drawn over the tail portion 18 of the elongated member 10. Other attachment means between the hollow member 30 and the elongated member 10 are possible, however. The rear end 17 of the elongated member 10 in this embodiment variant is flattened in order to facilitate the insertion of the elongated member 10 into the blood vessel and the removal of the elongated member 20 from the blood vessel after the surgical operation. In this embodiment variant, the fluid is dispensed to the tissues surrounding the blood vessel through apertures 32 on the hollow member 30.

Although the present disclosure has been described with reference to particular means, materials and embodiments, one skilled in the art can easily ascertain from the foregoing description the essential characteristics of the present disclosure, while various changes and modifications may be made to adapt the various uses and characteristics, without departing from the scope of the present invention, as set forth in the following claims.

## Claims

1. Surgical apparatus (100) for removing a blood vessel from a patient's body, comprising
an elongated member (10), having a front end (16) for inserting the elongated member (10) into, and pushing it through, the blood vessel,
a rear end (17) for pulling the elongated member (10) out of the blood vessel,
a hollow member (30) for conducting a fluid, which hollow member (30) is attached to the rear end (17) of the elongated member (10), and
at least one aperture (21, 32) in fluid connection with the hollow member (30) provided on the surgical apparatus (100) for dispensing the fluid during the removal of the blood vessel, **characterised in that** the front end (16) is tapered laterally and arranged to perforate through a wall of the blood vessel.

2. Surgical apparatus (100) according to claim 1, **characterised in that** the at least one aperture (32) for dispensing the fluid during the removal of the blood vessel is provided on the hollow member (30).

3. Surgical apparatus (100) according to claims 1 or 2, **characterised in that** at least one interface element (33) for connecting a fluid reservoir and filling the fluid into the hollow member (30) is provided at the hollow member (30).

4. Surgical apparatus (100) according to any one of the claims 1 to 3, **characterised in that** the front end (16) of the elongated member (10) is curved.

5. Surgical apparatus (100) according to any one of the claims 1 to 4, **characterised in that** an orifice (15) is provided on the front end (16) of the elongated member (10).

6. Surgical apparatus (100) according to any one of the claims 1 to 5, **characterised in that** the elongated member (10) comprises at least one curve (12, 13).

7. Surgical apparatus (100) according to any one of the claims 1 to 6, **characterised in that** the surgical apparatus (100) comprises a projecting member (20), the blood vessel being crumpled by the projecting member (20) during insertion and pushing through of the elongated member (20) in the blood vessel.

8. Surgical apparatus (100) according to any one of the claims 1 to 7, **characterised in that** the at least one aperture (21) is provided on the projecting member (20).

9. Surgical apparatus (100) according to any one of the claims 1 to 8, **characterised in that** the projecting member (20) comprises at least one fluid channel (31) for fluid connection between the hollow member (30) and the at least one aperture (21).

10. Surgical apparatus (100) according to any one of the claims 1 to 9, **characterised in that** the cross section of the projecting member (20) perpendicular to the insertion direction has a substantially oval form.

11. Surgical apparatus (100) according to any one of the claims 1 to 10, **characterised in that** the cross section of the projecting member (20) parallel to the insertion direction has a substantially curved and/or tapered form.

12. Surgical apparatus (100) according to any one of the claims 1 to 11, **characterised in that** it is made at least partially of plastic.

13. Surgical apparatus (100) according to any one of the claims 1 to 12, **characterised in that** the fluid is an anaesthetic, a saline solution, a vasoconstrictive agent, and/or a combination of these.

## Patentansprüche

1. Chirurgische Vorrichtung (100) zum Entfernen eines Blutgefässes aus dem Körper eines Patienten, umfassend
ein längliches Element (10), welches ein vorderes Ende (16) aufweist, um das längliche Element (10) in das Blutgefäss einzuführen und durch dasselbe zu schieben,
ein hinteres Ende (17), um das längliche Element (10) aus dem Blutgefäss herauszuziehen,
ein hohles Element (30), um eine Flüssigkeit zu leiten, wobei das hohle Element (30) an dem hinteren Ende (17) des länglichen Elements (10) angebracht ist, und
wenigstens eine Öffnung (21, 32), welche in Strömungsverbindung mit dem an der chirurgischen Vorrichtung (100) vorgesehenen hohlen Element (30) steht, um die Flüssigkeit beim Entfernen des Blutgefässes abzugeben,
**dadurch gekennzeichnet, dass** das vordere Ende (16) seitlich konisch zulaufend und so angeordnet ist, dass es eine Wand des Blutgefässes durchbohrt.

2. Chirurgische Vorrichtung (100) nach Anspruch 1, **dadurch gekennzeichnet, dass** die wenigstens eine Öffnung (32) zum Abgeben der Flüssigkeit beim Entfernen des Blutgefässes an dem hohlen Element (30) vorgesehen ist.

3. Chirurgische Vorrichtung (100) nach Anspruch 1 oder 2, **dadurch gekennzeichnet, dass** wenigstens ein Schnittstellenelement (33) zum Anschliessen eines Flüssigkeitsbehälters und zum Einfüllen der Flüssigkeit in das hohle Element (30) an dem hohlen Element (30) vorgesehen ist.

4. Chirurgische Vorrichtung (100) nach einem der Ansprüche 1 bis 3, **dadurch gekennzeichnet, dass** das vordere Ende (16) des länglichen Elements (10) gekrümmt ist.

5. Chirurgische Vorrichtung (100) nach einem der Ansprüche 1 bis 4, **dadurch gekennzeichnet, dass** ein Durchlass (15) an dem vorderen Ende (16) des länglichen Elements (10) vorgesehen ist.

6. Chirurgische Vorrichtung (100) nach einem der Ansprüche 1 bis 5, **dadurch gekennzeichnet, dass** das längliche Element (10) wenigstens eine Biegung (12, 13) umfasst.

7. Chirurgische Vorrichtung (100) nach einem der Ansprüche 1 bis 6, **dadurch gekennzeichnet, dass** die chirurgische Vorrichtung (100) ein hervorstehendes Element (20) umfasst, wobei das Blutgefäss von dem hervorstehenden Element (20) faltig zusammengedrückt wird, während das längliche Element (20) in das Blutgefäss eingeführt und durch dasselbe hindurch geschoben wird.

8. Chirurgische Vorrichtung (100) nach einem der Ansprüche 1 bis 7, **dadurch gekennzeichnet, dass** die wenigstens eine Öffnung (21) auf dem hervorstehenden Element (20) vorgesehen ist.

9. Chirurgische Vorrichtung (100) nach einem der Ansprüche 1 bis 8, **dadurch gekennzeichnet, dass** das hervorstehende Element (20) wenigstens einen Flüssigkeitskanal (31) zur Strömungsverbindung zwischen dem hohlen Element (30) und der wenigstens einen Öffnung (21) umfasst.

10. Chirurgische Vorrichtung (100) nach einem der Ansprüche 1 bis 9, **dadurch gekennzeichnet, dass** der Querschnitt des hervorstehenden Elements (20), der senkrecht zu der Einführungsrichtung verläuft, eine im Wesentlichen ovale Form aufweist.

11. Chirurgische Vorrichtung (100) nach einem der Ansprüche 1 bis 10, **dadurch gekennzeichnet, dass** der Querschnitt des hervorstehenden Elements (20), der parallel zu der Einführungsrichtung verläuft, eine im Wesentlichen gekrümmte und/oder konisch zulaufende Form aufweist.

12. Chirurgische Vorrichtung (100) nach einem der Ansprüche 1 bis 11, **dadurch gekennzeichnet, dass** sie wenigstens zum Teil aus Kunststoff hergestellt ist.

13. Chirurgische Vorrichtung (100) nach einem der Ansprüche 1 bis 12, **dadurch gekennzeichnet, dass** es sich bei der Flüssigkeit um ein Anästhetikum, eine Kochsalzlösung, ein gefässverengendes Mittel und/oder eine Kombination aus diesen handelt.

## Revendications

1. Appareil chirurgical (100) pour enlever un vaisseau sanguin du corps d'un patient, comprenant
un élément allongé (10) ayant une extrémité avant (16) pour insérer l'élément allongé (10) dans et le pousser à travers le vaisseau sanguin,
une extrémité arrière (17) pour extraire l'élément allongé (10) du vaisseau sanguin,
un élément creux (30) pour conduire un fluide, lequel élément creux (30) est relié à l'extrémité arrière (17) de l'élément allongé (10), et
au moins une ouverture (21, 32) en connexion fluidique avec l'élément creux (30) prévue sur l'appareil chirurgical (100) pour distribuer le fluide au cours de l'enlèvement du vaisseau sanguin, **caractérisé en ce que** l'extrémité avant (16) est amincie latéralement et agencée pour perforer à travers une paroi du vaisseau sanguin.

2. Appareil chirurgical (100) selon la revendication 1, **caractérisé en ce que** l'au moins une ouverture (32) pour distribuer le fluide au cours de l'enlèvement du vaisseau sanguin est prévue sur l'élément creux (30).

3. Appareil chirurgical (100) selon la revendication 1 ou 2, **caractérisé en ce qu'**au moins un élément d'interface (33) pour connecter un réservoir de fluide et verser le fluide dans l'élément creux (30) est prévu au niveau de l'élément creux (30).

4. Appareil chirurgical (100) selon l'une quelconque des revendications 1 à 3, **caractérisé en ce que** l'extrémité avant (16) de l'élément allongé (10) est courbe.

5. Appareil chirurgical (100) selon l'une quelconque des revendications 1 à 4, **caractérisé en ce qu'**un orifice (15) est prévu sur l'extrémité avant (16) de l'élément allongé (10).

6. Appareil chirurgical (100) selon l'une quelconque des revendications 1 à 5, **caractérisé en ce que** l'élément allongé (10) comprend au moins une cambrure (12, 13).

7. Appareil chirurgical (100) selon l'une quelconque des revendications 1 à 6, **caractérisé en ce que** l'appareil chirurgical (100) comprend un élément saillant (20), le vaisseau sanguin étant enchevêtré par l'élément saillant (20) au cours de l'insertion et de l'enfoncement de l'élément allongé (20) dans le vaisseau sanguin.

8. Appareil chirurgical (100) selon l'une quelconque des revendications 1 à 7, **caractérisé en ce que** l'au moins une ouverture (21) est prévue sur l'élément saillant (20).

9. Appareil chirurgical (100) selon l'une quelconque des revendications 1 à 8, **caractérisé en ce que** l'élément saillant (20) comprend au moins un canal fluidique (31) pour la connexion fluidique entre l'élément creux (30) et l'au moins une ouverture (21).

10. Appareil chirurgical (100) selon l'une quelconque des revendications 1 à 9, **caractérisé en ce que** la section transversale de l'élément saillant (20) perpendiculaire à la direction d'insertion possède une forme essentiellement ovale.

11. Appareil chirurgical (100) selon l'une quelconque des revendications 1 à 10, **caractérisé en ce que** la section transversale de l'élément saillant (20) parallèle à la direction d'insertion possède une forme essentiellement courbe et/ou amincie.

12. Appareil chirurgical (100) selon l'une quelconque des revendications 1 à 11, **caractérisé en ce qu'**il est fabriqué au moins partiellement en plastique.

13. Appareil chirurgical (100) selon l'une quelconque des revendications 1 à 12, **caractérisé en ce que** le fluide est un anesthésiant, une solution saline, un agent vasoconstricteur et/ou une combinaison de ceux-ci.
